# EUROPEAN PATENT SPECIFICATION

(11) **EP 0 321 912 B1**
(45) Date of publication and mention of the grant of the patent: **15.02.1995**
(21) Application number: 88121260.9
(22) Date of filing: 19.12.1988
(51) Int. Cl.: A61F 2/06, A61M 29/00

(54) **Removable endo-arterial devices intended to repair detachments in arterial walls**
Entfernbare endoarterielle-Vorrichtung, bestimmt für die Reparatur von Ablösungen in arteriellen Wänden
Dispositifs endo-artériels amovibles destinés à réparer les décollements de parois des artères

(30) Priority: 18.12.1987 FR 8717975
(43) Date of publication of application: 28.06.1989
(73) Proprietor: Delsanti, Gerard L, F-13390 Auriol (FR)
(72) Inventor: Delsanti, Gerard L, F-13390 Auriol (FR)
(74) Representative: Baillie, Iain Cameron

(56) References cited:
- EP-A- 0 183 372
- EP-A- 0 186 267
- EP-A- 0 274 846
- DE-A- 3 532 653
- FR-A- 2 454 293
- US-A- 1 677 671
- US-A- 4 650 466

## Description

### Technical Field

The present invention provides endo-arterial devices for temporary installation in an artery in order to re-attach flaps which have been detached from the wall according to the preamble of claim 1. Such a device is known from US-A-4 650 466.

The technical field of the invention is, thus, the construction of surgical equipment used in cardio-vascular interventions.

### Background of the Invention

There are known devices consisting of a small inflatable balloon at the end of a catheter used to dilate strictures in the arteries, especially the coronary arteries.

Such a catheter bearing a balloon is introduced into an artery, for example into the femoral artery, until the balloon reaches the stricture. The balloon is then inflated with a fluid pumped in through the catheter and pushes back the arterial wall, thus eliminating the stricture. The balloon must then be deflated very quickly, since it blocks the artery and impairs blood circulation.

It so happens that a similar intervention may cause detachments of the part of the arterial wall called intima, and the detached wall flaps inhibit the blood circulation and may result in severe medical complications and even be fatal if circulation is interrupted.

Devices consisting of a cylindrical elastic cuff inserted over an inflatable balloon fixed to the end of a catheter have been tried for the prevention of such accidents. The balloon is folded back over the cuff so as to keep the latter in an elongated shape of small diameter while it is being pushed through the arteries. Once the balloon bearing the elastic cuff has arrived at the site of the wall detachment, it is inflated so that the folded part slips loose releasing the cuff which increases in diameter and plasters itself against the internal wall of the artery where it remains indefinitely. This device has the negative feature of introducing a foreign body into the artery to remain stationary there, and presents risks of blood clots to the patient.

One invention of interest is disclosed in U.S. Patent No. 4,650,466 issued to Ronald B. Luther. In this patent, an angioplasty device is disclosed comprising a woven tube of metal or plastic fibers and a retraction stylet that is attached at one end to a catheter tube for insertion into a vein, artery, and the like. The retraction stylet comprised of a plurality of wires, is pulled back and forth to enlarge and then elongate the woven portion of the tube. In this way, plaque and similar material may be removed from the vein, artery, etc.

French Patent No. 7 910 971 discloses a similar device which is used in conjunction with a guidewire and has a distendable end piece.

### Brief Summary of the Invention

The invention is as disclosed in the accompanying claims.

It comprises a removable endo-arterial device for repairing detachments of an arterial wall, said device having a deformable cuff made of a netting of plaited and interlocked wires which is mounted at the end of a catheter and adapted to be introduced into an artery, said device also including means activated from the external end of said catheter to bring two ends of said deformable cuff closer together or move them further apart in order to give said device a wider shape which applies said cuff against the arterial wall or an elongated shape which permits said cuff and the catheter to be introduced into the artery or withdrawn therefrom, said device including an axial conduit for receipt of a guidewire which conduit enables the unobstructed gliding of the guidewire through said axial conduit, said axial conduit defining an axial conduit in the distal end of said cuff.

The invention permits the deformable cuff to be passed over a guide wire which has been previously placed in an artery.

According to a preferred embodiment of the invention, the means used to reduce or extend the distance between the two ends of the deformable cuff are made up by a wire of the piano wire type which makes it possible to exert a push and which is fixed to the distal end of the cuff while freely passing through the proximal end of it and extending through the entire length of the catheter.

According to another embodiment, a device according to the present invention includes, in addition, an inflatable balloon located inside the deformable cuff and is mounted at the end of an inflation tube which, in turn, runs inside the catheter.

The invention provides new devices usable in cardio-vascular interventions, especially in interventions intended to remove stenoses of the coronary arteries in case of a severe risk of infarct or after an infarct has occurred.

Devices according to the present invention have the advantage that the deformable net allows the blood to pass between its mesh openings when it is applied against the wall of an artery. It can therefore be left in place for a duration on the order of one or more hours, which is more than sufficient to ensure cicatrization of the flaps detached from the arterial wall.

As compared to the known devices which involve an elastic cuff remaining stationary in the artery, devices according to the present invention have the advantage of being removable so that there is no risk of rejection phenomena or of the formation of blood clots. The devices according to the present invention may include an inflatable balloon placed inside a cuff or deformable net, making it possible to treat a stenosis and, if necessary, to immediately repair the arterial wall. They therefore reduce the risk of postoperative complications and can even be used for interventions on strictures of the common trunk of the coronary arteries.

### Description of the Drawings

The following detailed description refers to the enclosed drawings which represent examples of embodiments of the devices accordingly to the present invention without being in any way limitative.

Fig. 1 is a longitudinal section of a first embodiment of a device according to the present invention.

Fig. 2 is a longitudinal section of the fixation of the proximal end of the deformable cuff over the distal end of the catheter.

Fig. 3 is a longitudinal section of a second embodiment of a device according to the present invention.

Fig. 4 is a cross section along IV-IV in Fig. 3.

### Detailed Description

During a first intervention, a catheter bearing an inflatable balloon at the end, was inserted into an artery presenting a stricture or stenosis, until its balloon reached the stenosis. The balloon was then inflated with a fluid pumped in through the catheter. The inflated balloon pushed the arterial wall back and removed the stenosis. Since an inflated balloon blocks the artery, it has to be quickly deflated.

The inflating and deflating operation of the balloon may have been repeated several times. Subsequently, the balloon and catheter were withdrawn. During these operations, the internal wall of the artery, called intima, suffered some detachments which, if not repaired, could block the artery and result in the death of the patient.

When an inflatable balloon is introduced into an artery to correct a stenosis, the intervention generally begins with insertion into the artery of a guide wire of piano-wire type. The catheter is then slipped over this wire bearing at its end the inflatable balloon through which passes a small axial tube which engages the wire and follows it.

When the stenosis has been eliminated, the balloon is deflated and withdrawn from the artery, but the guide wire may be left in place for some minutes in case it becomes necessary to use the balloon again.

A device according to the present invention is depicted in the Figures and it can be advantageously used to facilitate repair of the detachments. It includes a removable endo-arterial prothesis intended to reduce the risks caused by the detachments by applying the detached flaps of wall against the artery long enough for cicatrization to take place.

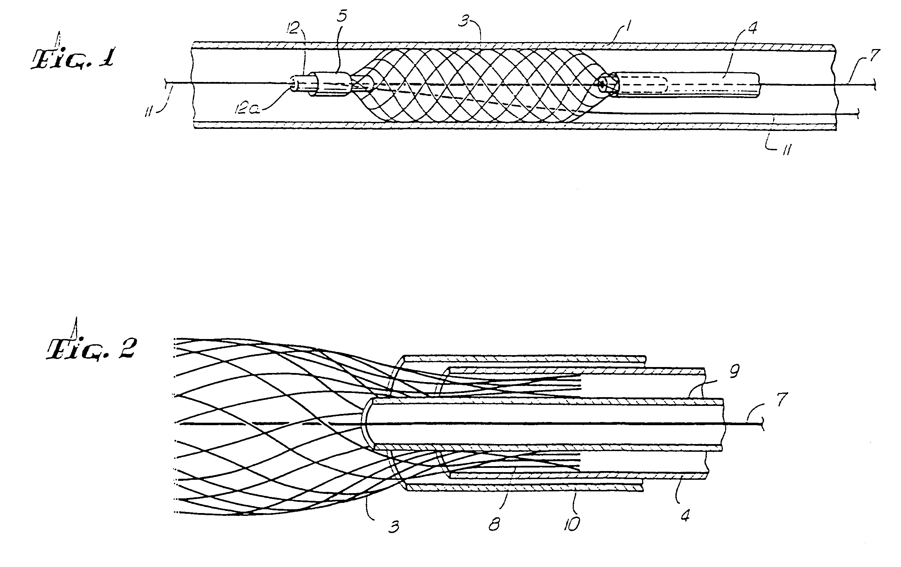

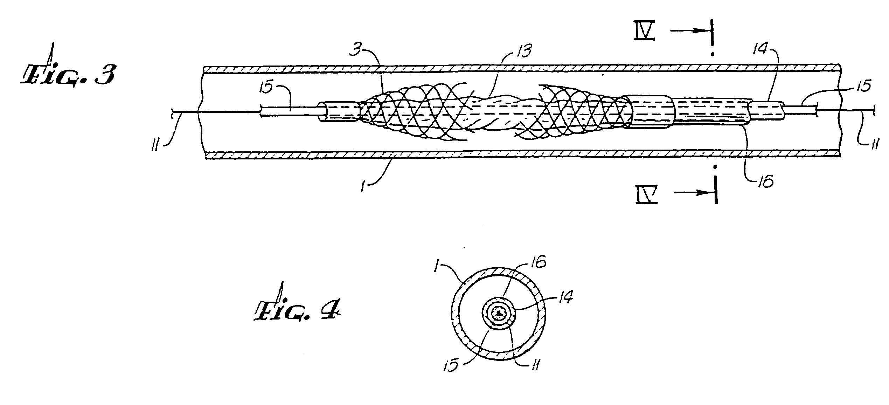

The device of Figs. 1 and 2 includes a deformable cuff 3 made of a net of twisted and interlocked wires which could, for instance, be of stainless steel wires or of any other material having equivalent properties of compatibility with the blood. The deformable cuff 3 is fixed to the end of a small flexible tube 4 which has a diameter on the order of a few millimeters and serves as catheter. The distal end of the deformable cuff 3 is fixed to a small muff 5.

A device according to Figs. 1 and 2 includes, in addition, an axial wire 7 of the piano-wire type which is fixed to the distal end of cuff 3, passes through the latter and extends the entire length of the catheter 4.

The catheter is preferably equipped at its proximate end with a funnel of a known kind, for example, a funnel of the "LUER-LOCK" type. Wire 7 passes through the funnel, so that its end is accessible outside the catheter.

Fig. 2 is a large scale axial section view of the proximal end of the deformable cuff 3. In this Figure the flexible tube or catheter 4 and the axial wire 7 are clearly seen.

Wires 8 which make up the end of the deformable cuff 3 are unraveled, inserted and fixed parallel to the axis between the end of tube 4 and a second tube 9 which is placed inside the latter and in which wire 7 runs freely. Tube 9 should preferably extend over the entire length of tube 4. The ends of wires 8 are glued between tube 4 and tube 9. A thermo-shrinkable sleeve 10 is preferably slipped over the proximal end of the cuff and then heat-shrunk to it.

In Fig. 2 it can be seen that the proximal end of the deformable cuff 3, which is fixed to catheter 4 and to the internal tube 9, slides freely over pull wire 7.

The device according to Fig. 1 also includes a small piece of tubing 12 which provides an axial opening or conduit 12a passing through the distal end of the cuff. The numeral 11 represents a guide wire inserted into artery 1.

The practical application of a device according to Figs. 1 and 2 is the following:
The end of the guide wire 11 which extends outside the artery and the patient is inserted through conduit 12a and then is passed through the mesh of the net so that it lies along the outside of tube 4. This permits the cuff to be guided on guide wire 11 until it reaches the zone where the stenosis had been located and previously corrected with an inflatable balloon.

During this insertion, the net 3 is in an elongated position. Once the device has arrived at the site, the axial wire 7 is pulled to open the net 3 and bring it into the position shown in Fig. 1. The dilated cuff comes to rest against the arterial wall, thus pressing the detached flaps back against the wall. Cuff 3 is left in this position for as long as one or more hours, since the blood can freely circulate through the mesh openings of the cuff which, at that time, are open. When it is deemed that sufficient time has elapsed for the flaps to adhere again to the wall, the outer end of wire 7, which is stiff enough not to bend, is pushed, thereby causing the distal end of cuff 3 to move away, so that the cuff is again in its elongated position. Subsequently, the net is re-folded by pushing on the axial wire 7 and pulling on tube 4, and the catheter is withdrawn from the artery along guide wire 11.

Figs. 3 and 4 represent another embodiment of a device according to the invention, including a netted cuff combined with an inflatable balloon.

To this date, inflatable balloons are used to correct the stenoses of the coronary arteries but only downstream from the common trunk, i.e. from the bifurcation of the circumflex and interventricular anterior arteries. They are used only very exceptionally to intervene on the common trunk because of the fact that detachments of the wall in the common trunk occurring after the intervention with an inflatable balloon would deprive a large part of the heart of irrigation and thus cause almost instantaneous death.

Figs. 3 and 4 show a device according to this invention which would permit interventions on stenoses of the common trunk and also on strictures located below the latter or in other arteries.

The device according to Fig. 3 includes a deformable balloon 13 of the kind currently used for angioplasty mounted at the end of a flexible tube 14. An axial tube 15 passes through the balloon from one end to the other and is fixed to the latter by one or both of its ends.

Figs. 3 and 4 show an embodiment having two coaxial tubes 14 and 15. As a variation, a single tube divided into two conduits by an inner partition could alternatively be used.

Tubes 14 and 15 extend to the outside where they preferably end in a funnel of a known type, for example a "LUER-LOCK" funnel, which may be simple or include a derivation for the injection of fluid into the catheter. The axial tube 15 is adapted to receive the guide wire 11 which has previously been introduced into the artery. The interspace between tubes 14 and 15 is intended for injection or pumping of the inflation fluid into balloon 13.

The device also includes a net 3 in form of a cuff, compromising plaited wires surrounding the inflatable balloon, the distal end of which is fixed to the distal end of the balloon, while the proximal end slides freely on tube 14.

For the sake of clarity in the drawing, net 3 is shown partly cut away in Fig. 3. Net 3 is mounted at its proximal end to a flexible tube 16 which encloses tubes 14 and 15.

The steps of practical application are the following:
When a stenosis is to be corrected, a guide wire 11 is first introduced into the artery. The axial tube 15 is then inserted over it and the device according to Fig. 3 is then pushed along guidewire 11 in a stretched configuration,i.e. the balloon 13 is collapsed and net 3 is elongated. The progress is checked by radiography. Once the net and balloon are in place, fluid is pumped in between tubes 14 and 15 which inflates the balloon and, in turn, dilates the artery and eliminates the stricture.

The highly flexible and deformable net 3 does not hamper the inflation of the balloon, since it slides in relation to the latter. The inflation of the balloon causes the dilation of the net.

Once the stricture has been eliminated, the fluid is withdrawn and the balloon is deflated, but the net remains in place against the internal wall of the artery. If necessary, the net is pressed against the wall of the artery by pushing on tube 16 which is sufficiently rigid to transmit the thrust. The axial tube 15 is held fast to immobilize the distal end.

The blood circulates through the mesh of the netting and the prothesis may be left in this position for a time on the order of one to several hours which is more than enough for the eventual detachments of the inner arterial wall to heal.

Subsequently, tube 16 is pulled while keeping the axial tube 15 in place which has the effect of moving the two ends of net 3 further apart and putting the latter back into an elongated position, then the entire complex of the device is pulled out of the artery along guide wire 11.

In the embodiment according to Figs. 3 and 4, it is not necessary to use a wire to cause the deformation of cuff 3. The central tube 15 which is fixed to the distal end of the net and tube 16 which is fixed to the proximal end of the net are sufficient to permit moving these two ends toward or away from each other.

## Claims

1. A removable endo-arterial device for repairing detachments (2) of an arterial wall (1), said device having a deformable cuff (3) made of a netting of plaited and interlocked wires which is mounted at the end of a catheter (4) and adapted to be introduced into an artery (1), said device also including means (7, 13, 15, 16) activated from the external end of said catheter (4) to bring two ends of said deformable cuff (3) closer together or move them further apart in order to give said device a wider shape which applies said cuff (3) against the arterial wall (1) or an elongated shape which permits said cuff (3) and the catheter (4) to be introduced into the artery or withdrawn therefrom, said device being characterized in that it includes an axial conduit (12, 15) for receipt of a guidewire (11) which conduit enables the unobstructed gliding of the guidewire (11) through said conduit (12, 15), said axial conduit (12, 15) defining an axial conduit in the distal end of said cuff (3).

2. The device of claim 1 further characterized in that said means to bring the two ends of said deformable cuff (3) closer together or to space them further apart is comprised of wire (7) of the piano-wire type which is fixed to a distal end of said cuff (3) while freely running through the proximal end of said cuff (3) and extending through the entire length of the catheter.

3. The device of claim 1 further characterized in that said means to bring the ends of said cuff (3) closer together is an inflatable balloon (13) mounted inside said cuff (3) at the end of an inflation tube (14) which lies within catheter (16) which carries deformable cuff (3).

4. The device of claim 3 further characterized by an axial guide tube (15) which passes through said balloon (13), said guide tube (15) being fixed to the distal end of said balloon (13) and extending over the entire length of said catheter (16).

5. The device of either one of claims 3 or 4 further characterized in that said distal end of said balloon (13) and said deformable cuff (3) are fixed while the proximal end of said deformable cuff (3) slides freely on said inflation tube (14).

6. The device of any one of the preceding claims further characterized in that said cuff (3) has an open proximal end.

7. The device of any one of claims 3 through 5 further characterized in that the interior of said inflatable balloon (13) is in fluid communication with the lumen extending through said catheter (4, 16).

8. The device of claim 1 further characterized in that the arrangement is such that during use of the device said guidewire (11) exits through the mesh openings of the netting of said cuff (3).

## Patentansprüche

1. Entfernbare endoarterielle Vorrichtung zur Reparatur von Ablösungen (2) an einer Arterienwand (1), wobei die Vorrichtung eine verformbare Manschette (3) aus einem Netz geflochtener und ineinander verschlungener Drähte aufweist, die am Ende eines Katheters (4) angebracht ist und in eine Arterie (1) eingeführt werden kann, wobei die Vorrichtung außerdem Einrichtungen (7, 13, 15, 16) umfaßt, die vom äußeren Ende des Katheters (4) aus betätigt werden, um zwei Enden der verformbaren Manschette (3) näher zusammen zu bringen oder weiter auseinander zu bewegen, damit die Vorrichtung eine breitere Form bekommt, die die Manschette (3) an der Arterienwand (1) anliegen läßt, oder eine langgestreckte Form, aufgrund der die Manschette (3) und der Katheter (4) in die Arterie eingeführt oder aus dieser entfernt werden können, wobei die Vorrichtung dadurch gekennzeichnet ist, daß sie ein axiales Röhrchen (12, 15) zur Aufnahme eines Führungsdrahtes (11) umfaßt, welches ein ungehindertes Gleiten des Führungsdrahtes (11) durch das Röhrchen (12, 15) ermöglicht, wobei das axiale Röhrchen (12, 15) ein axiales Röhrchen im distalen Ende der Manschette (3) darstellt.

2. Vorrichtung nach Anspruch 1, des weiteren dadurch gekennzeichnet, daß die Einrichtung, die die beiden Enden der verformbaren Manschette (3) näher zusammen bringt oder weiter voneinander entfernt, aus Draht (7) in der Art eines Klavierdrahtes besteht, der am distalen Ende der Manschette (3) befestigt ist, während er ungehindert durch das proximale Ende der Manschette (3) verläuft und sich durch die gesamte Länge des Katheters erstreckt.

3. Vorrichtung nach Anspruch 1, des weiteren dadurch gekennzeichnet, daß die Einrichtung, die die Enden der Manschette (3) näher zusammen bringt, ein aufblasbarer Ballon (13) ist, der im Inneren der Manschette (3) am Ende eines Aufblasschlauches (14) angebracht ist, der im Inneren des Katheters (16) liegt, der die verformbare Manschette (3) trägt.

4. Vorrichtung nach Anspruch 3, des weiteren gekennzeichnet durch einen axialen Führungsschlauch (15), der durch den Ballon (13) verläuft, wobei der Führungsschlauch (15) am distalen Ende des Ballons (13) befestigt ist und sich über die gesamte Länge des Katheters (16) erstreckt.

5. Vorrichtung nach einem der Ansprüche 3 oder 4, des weiteren dadurch gekennzeichnet, daß das distale Ende des Ballons (13) und die verformbare Manschette (3) feststehen, während das proximale Ende der verformbaren Manschette (3) sich frei auf dem Aufblasschlauch (14) bewegen kann.

6. Vorrichtung nach einem der vorhergehenden Ansprüche, des weiteren dadurch gekennzeichnet, daß die Manschette (3) ein offenes proximales Ende besitzt.

7. Vorrichtung nach einem der Ansprüche 3 bis 5, des weiteren dadurch gekennzeichnet, daß das Innere des aufblasbaren Ballons (13) in Flüssigverbindung mit dem Lumen des Katheters (4, 16) steht.

8. Vorrichtung nach Anspruch 1, des weiteren dadurch gekennzeichnet, daß die Anordnung dergestalt ist, daß im Gebrauch der Vorrichtung der Führungsdraht (11) durch die Maschenöffnungen im Netz der Manschette (3) herausragt.

## Revendications

1. Dispositif endo-artériel amovible pour réparer les décollements (2) d'une paroi artérielle (1), ledit dispositif étant muni d'une manchette déformable (3) faite d'un filet de fils tressés et enchevêtrés placé à l'extrémité d'un catéther (4) et adapté pour être introduit dans une artère (1), ledit dispositif incluant également des moyens (7, 13 ,15, 16) actionnés à partir de l'extrémité externe dudit cathéter (4) pour rapprocher deux extrémités de ladite manchette déformable (3) ou les éloigner l'une de l'autre afin de donner audit dispositif une forme plus élargie qui applique ladite manchette (3) contre la paroi artérielle (1) ou une forme plus allongée qui permet d'introduire ladite manchette (3) et le cathéter (4) dans l'artère ou de les en retirer, ledit dispositif étant caractérisé en ce qu'il inclut un conduit axial (12, 15) permettant de recevoir un fil de guidage (11), ledit conduit permettant le glissement sans obstruction du fil de guidage (11) à travers ledit conduit (12, 15), ledit conduit axial (12, 15) définissant un conduit axial dans l'extrémité distale de ladite manchette (3).

2. Dispositif de la revendication 1 caractérisé en outre en ce que ledit moyen servant à rapprocher ou à éloigner les deux extrémités de ladite manchette (3) déformable est composé d'un fil (7) du type corde à piano, fixé à l'extrémité distale de ladite manchette (3) tout en passant librement à travers l'extrémité proximale de ladite manchette (3) et s'étendant sur toute la longueur du catéther.

3. Dispositif selon la revendication 1 caractérisé en outre en ce que ledit moyen pour rapprocher les extrémités de ladite manchette (3) est un ballonnet gonflable (13) placé à l'intérieur de ladite manchette (3) à l'extrémité d'un tuyau de gonflage (14) situé au sein du cathéter (16) qui porte ladite manchette déformable (3).

4. Procédé selon la revendication 3 caractérisé en outre par un tube de guidage axial (15) qui passe à travers ledit ballonnet (13), ledit tube de guidage (15) étant fixé à l'extrémité distale dudit ballonnet (13) et s'étendant sur la longueur totale dudit cathéter (16).

5. Dispositif selon l'une quelconque des revendications 3 ou 4 caractérisé en outre en ce que l'extrémité distale dudit ballon (13) et de ladite manchette déformable (3) sont fixées alors que l'extrémité proximale de ladite manchette déformable (3) glisse librement sur ledit tuyau de gonflage (14).

6. Dispositif selon l'une quelconque des revendications précédentes caractérisé en outre en ce que ladite manchette (3) a son extrémité proximale ouverte.

7. Dispositif selon l'une quelconque des revendications 3 à 5 caractérisé en outre en ce que l'intérieur dudit ballonnet gonflable (13) est en communication fluidique avec le canal s'étendant à travers ledit cathéter (4, 16).

8. Dispositif selon la revendication 1 caractérisé en outre en ce que l'agencement est tel durant l'utilisation du dispositif que ledit fil de guidage (11) passe à travers les ouvertures de maillage de ladite manchette (3).
